# FASCICULE DE BREVET EUROPEEN

(11) **EP 2 755 601 B1**
(45) Date de publication et mention de la délivrance du brevet: **10.04.2019**
(21) Numéro de dépôt: 12759157.6
(22) Date de dépôt: 17.09.2012
(51) Int. Cl.: A61F 2/16

(54) **DISPOSITIF POUR INJECTER UNE LENTILLE INTRAOCULAIRE DANS UN OEIL**
VORRICHTUNG ZUM EINSETZEN EINER INTRAOKULARLINSE IN DAS AUGE
DEVICE FOR INJECTING AN INTRAOCULAR LENS INTO AN EYE

(30) Priorité: 16.09.2011 FR 1158252
(43) Date de publication de la demande: 23.07.2014
(73) Titulaire: Medicontur Orvostechnikai Korlátolt Felelösségü Társaság, 2072 Zsámbék (HU)
(72) Inventeur: KONTUR, László Ferenc, 1224 Budapest (HU); TURKEVI-NAGY, Nándor, 2072 Zsámbék (HU); STEFAN, Attila, 2089 Telki (HU)
(74) Mandataire: Petit, Maxime
(86) Numéro de dépôt international: PCT/EP2012/068277
(87) Numéro de publication internationale: WO 2013/038021

(56) Documents cités:
- DE-U1- 20 221 644
- DE-U1- 20 221 644
- US-A1- 2003 036 765
- US-A1- 2008 058 830
- US-A1- 2009 125 034

## Description

L'invention concerne les dispositifs d'injection d'une lentille intraoculaire, ci-après IOL, dans un oeil.

Il est aujourd'hui courant d'injecter dans l'oeil des IOL pliables souples fabriquées dans des matériaux tels que la silicone, les acryliques souples ou les hydrogels, ce qui permet de ne pratiquer dans l'oeil qu'une incision auto-suturante de l'ordre de 3mm lors de la mise en place de l'IOL.

A cette fin de nombreux dispositifs d'injection ont été proposés. Ils sont en général constitués d'une cartouche dans laquelle l'IOL est repliée et qui est adaptée sur un injecteur de type seringue dont l'extrémité de sortie est conformée en canule de faible diamètre, le poussoir de l'injecteur forçant l'expulsion de l'IOL à travers ladite canule.

Pour assurer que l'IOL se plie correctement sans risque de déformation ou de pincement, divers dispositifs de cartouches perfectionnées ont été conçus dont un exemple est décrit dans le document FR 2 808 993. Selon ce document, la cartouche est agencée de manière à former un module de pliage qui comprend deux mors mobiles l'un par rapport à l'autre, chaque mors comprenant une paroi de pliage constituée par une portion de surface cylindrique et une paroi plane solidaire de la paroi de pliage, les deux parois planes étant disposées dans des plans parallèles. Un axe de rotation orthogonal aux parois planes contrôle le mouvement relatif de rotation d'un mors par rapport à l'autre. L'un des mors peut occuper trois positions par rapport à l'autre : une première position dans laquelle les parois planes sont décalées l'une par rapport à l'autre, l'IOL étant mise en place sur la paroi plane inférieure, une seconde position dans laquelle les parois planes sont en regard l'une de l'autre, les paroi planes et les parois de pliage définissant un volume de confinement de l'implant, et une troisième position dans laquelle les parois de pliage sont raccordées entre elles pour définir un volume sensiblement cylindrique formant une chambre de pliage. Le module de pliage est conçu pour constituer une chambre de stockage de l'IOL à l'état non contraint avant son utilisation et être adapté sur l'injecteur au moment de ladite utilisation. Cependant, il impose au chirurgien de procéder au pliage de l'IOL en agissant sur les mors mobiles du module de pliage avant de procéder à l'injection. De plus, l'injecteur dans son ensemble doit être stérilisé et stocké dans un emballage contenant un liquide de stockage tel que de l'eau pure ou une solution aqueuse saline. De ce fait, les emballages sont encombrants et relativement lourds, donc coûteux à expédier, et lorsque le chirurgien les ouvre pour utiliser un injecteur ce dernier est mouillé ce qui rend sa manipulation incommode. En outre, malgré la stérilisation, il subsiste un danger de contamination par des pyrogènes généré par des impuretés résultant de l'assemblage de nombreuses surfaces complexes. Enfin, la lubrification de la chambre de compactage et d'éjection qui fait partie intégrante de la cartouche n'est pas optimale parce qu'à ce jour il n'est pas connu de traitement de surface ou de lubrifiant qui soit stable pendant la stérilisation à la vapeur et/ou un bain prolongé dans le liquide de stockage.

Le document BE 1016 692 décrit un autre dispositif d'injection qui supprime l'opération manuelle de compactage (pliage ou enroulement) préalable à l'injection, ce compactage de l'IOL étant produit par l'action du poussoir de l'injecteur. Selon ce document, la cartouche dans laquelle l'IOL est stockée à l'état non contraint est conformée en un entonnoir se raccordant à une canule de sortie. Elle est pourvue de moyens de retenue qui assurent son maintien dans un récipient de stockage et de moyens d'accrochage sur l'extrémité libre de l'injecteur. Il suffit au chirurgien, après avoir ouvert le récipient contenant la cartouche, d'encliqueter la cartouche sur l'injecteur et d'agir sur le poussoir

Cette conception permet de résoudre un certain nombre des inconvénients visés ci-dessus du fait que le corps d'injecteur est emballé dans un emballage sec stérilisé par le gaz oxyde d'éthylène (EtO) et que, par conséquent il est sec lorsque le chirurgien le prend en main. De plus l'emballage est plus léger. Cependant, la cartouche préchargée reste emballée, stérilisée et stockée dans un emballage humide contenant un liquide de stockage, ce qui laisse subsister le problème de la stabilité de son revêtement de surface et/ou de sa lubrification. Pour cette raison, au moment de son utilisation, la cartouche contenant l'IOL préchargée doit être soumise à des rinçages multiples avec une solution saline équilibrée pour éviter les inflammations post-opératoires.

On connait du document US 2009/0125034 un injecteur d'IOL préchargé. La présente invention vise à remédier à cet inconvénient et à procurer un dispositif d'injection pour lentille intraoculaire pliable dans lequel la nécessité des rinçages préalables est supprimée et qui est fiable. L'idée à la base de l'invention est notamment de dissocier la cartouche de stockage de l'IOL de la chambre de compactage et d'éjection qui peut dès lors être soumise à une stérilisation par gaz d'oxyde d'éthylène assurant la stabilité de son revêtement de surface et/ou de sa lubrification. A cet effet, l'invention a pour objet un dispositif d'injection pour lentille intraoculaire souple comprenant un corps d'injecteur logeant un poussoir coulissant et une chambre de compactage et d'éjection solidaire dudit corps d'injecteur, caractérisé en ce que la chambre de compactage et d'injection est articulée sur l'extrémité distale du corps d'injecteur de manière à pouvoir prendre deux positions, d'une part, une position dite de chargement dans laquelle au moins une partie est repliée le long du corps d'injecteur de manière décalée transversalement par rapport à l'axe longitudinal du corps d'injecteur de façon à permettre le chargement, dans le corps d'injecteur, suivant ledit axe longitudinal, d'une cartouche contenant une lentille intraoculaire à l'état non contraint et, d'autre part, une position dite d'injection dans laquelle la chambre de compactage et d'injection est verrouillée dans le prolongement dudit corps d'injecteur.

Ce dispositif d'injection selon l'invention permet, en repliant au moins une partie de la chambre de compactage et d'éjection (partie fonctionnelle articulée qui participe au compactage et à l'injection de la lentille) le long du corps d'injecteur (contre celui-ci), de laisser libre la place dans le corps d'injecteur pour y charger une cartouche (qui était préalablement stockée à part dans un récipient stérile) dans le prolongement dudit corps.

La partie fonctionnelle (mobile) de la chambre de compactage et d'éjection étant écartée latéralement par rapport à l'axe longitudinal du corps (disposée de façon adjacente au corps d'injecteur), elle ne participe pas à l'opération de chargement de la cartouche sur le corps, opération qui est susceptible d'induire des efforts mécaniques sur les éléments impliqués dans l'opération. Si la partie fonctionnelle de la chambre de compactage et d'éjection participait à cette opération de chargement elle serait affectée par de tels efforts mécaniques. Elle pourrait alors subir des déformations de nature à perturber l'opération ultérieure de compactage et d'éjection de la lentille (ex : défaut d'alignement de l'axe d'insertion de la lentille lors de cette opération) avec tous les risques et conséquences qu'il est possible d'imaginer. Par ailleurs, l'axe d'articulation de la chambre ou d'une partie de celle-ci est orthogonal à l'axe longitudinal du corps d'injecteur et est décalé transversalement par rapport à cet axe. Ainsi, lorsque la chambre de compactage et d'éjection est verrouillée dans le prolongement du corps d'injecteur, il n'y a pas de risque que la poussée exercée sur le poussoir coulissant crée un moment d'inertie propice à provoquer une rotation de l'ensemble cartouche-chambre (le verrouillage est en effet adapté à fournir un assemblage verrouillé de manière irréversible, ce qui augmente la fiabilité du dispositif). Le dispositif selon l'invention est donc particulièrement fiable. On notera que la chambre de compactage et d'éjection, ou la partie mobile de celle-ci, peut également être verrouillée dans la position de chargement.

Selon un mode préférentiel de réalisation, le dispositif d'injection pour lentille intraoculaire souple selon l'invention est caractérisé en ce que la partie distale du corps d'injecteur comporte une chambre de réception pour la cartouche et en ce que le corps d'injecteur porte un moyen de verrouillage dit alternatif qui est apte à occuper deux positions de blocage successives, à savoir une première position qui bloque le mouvement du poussoir lorsque la chambre de compactage et d'injection est dans la position de chargement d'une cartouche, puis une deuxième position qui bloque (verrouille) la chambre de compactage et d'éjection en position d'injection dans le prolongement du corps d'injecteur, et libère (déverrouille) instantanément le poussoir coulissant qui peut maintenant être actionné. Ainsi, un seul et même moyen de verrouillage est apte à assurer deux types de verrouillage différents (deux fonctions distinctes) pour les deux positions respectives consécutives de la chambre de compactage et d'éjection. Un utilisateur peut facilement, en manipulant le moyen de verrouillage, passer successivement, de façon irréversible, de la première position à la deuxième position de blocage, sans pouvoir retourner à la première position. Ce mécanisme participe à la sécurisation et à la fiabilité de l'utilisation du dispositif à un stade ultime de préparation (moyen de verrouillage armé) et garantit le caractère unique de son usage (« à usage unique »). En effet, une mauvaise préparation de l'injection (par exemple en raison d'un dispositif d'injection qui manque de fiabilité) peut induire un incident lors de l'injection, ce qui nécessitera de pratiquer une incision de plus grande ouverture afin de retirer l'implant endommagé.

Selon une caractéristique possible de l'invention, le moyen de verrouillage alternatif est apte à coulisser le long du corps d'injecteur pour passer de façon irréversible de la première position de blocage à la deuxième, ce qui permet un déplacement aisé du moyen de verrouillage par un simple mouvement de la part d'un utilisateur.

Selon une autre caractéristique possible de l'invention, le moyen de verrouillage alternatif comprend une portion de préhension pour un utilisateur et deux portions disposées aux deux extrémités opposées proximale et distale dudit moyen de verrouillage et qui sont respectivement aptes à coopérer avec le poussoir coulissant et avec la chambre de compactage et d'éjection (par exemple via des moyens d'encliquetage prévus sur la chambre) afin de verrouiller cette dernière de manière irréversible avec le corps de l'injecteur (la cartouche contenant la lentille est emprisonnée dans la chambre de compactage et d'éjection parfaitement dans l'axe de l'injection). Le moyen de verrouillage est ainsi de conception particulièrement simple et efficace. En raison de la position dégagée latéralement d'au moins une partie de la chambre de compactage et d'éjection (partie qui participe au compactage et à l'injection de la lentille) lors du chargement d'une cartouche, ladite au moins une partie de chambre n'est pas affectée.

Selon d'autres caractéristiques possibles de l'invention, prises isolément ou en combinaison l'une avec l'autre :
- le moyen de verrouillage alternatif présente la forme générale d'un cavalier muni de pattes destinées à guider le coulissement dudit cavalier dans des rainures latérales aménagées sur les cotés longitudinaux du corps d'injecteur ;
- le cavalier porte à sa partie proximale un pêne courbe coopérant avec une ouverture du poussoir;
- la partie distale du cavalier porte une languette qui s'étend en saillie vers l'avant et est destinée à s'engager (définitivement), dans la deuxième position de blocage du cavalier, sur une portion proximale de la chambre de compactage et d'éjection pour bloquer cette dernière en position fermée; à ce stade, seul le mouvement du poussoir coulissant en vue du compactage et de l'injection est possible ;
- près de son extrémité distale, la face inférieure de la languette du cavalier comporte une rainure destinée à coopérer avec des moyens d'encliquetage disposés sur la chambre de compactage et d'éjection ;
- la chambre de compactage et d'éjection comporte une partie proximale montée de manière fixe sur le corps d'injecteur et une partie distale qui est articulée par une charnière (ex : souple) à la partie proximale, l'axe de la charnière étant orthogonal par rapport à l'axe longitudinal du corps d'injecteur et décalé transversalement par rapport audit axe longitudinal ; la partie distale forme la partie fonctionnelle articulée de la chambre, tandis que la partie proximale qui est par exemple une monture sert de support mécanique à la partie distale pour son mouvement de rotation autour de l'axe de la charnière; selon une variante, la chambre est en une seule partie articulée par une charnière à l'extrémité distale du corps d'injecteur, l'axe de la charnière étant orthogonal par rapport à l'axe longitudinal du corps d'injecteur et décalé transversalement par rapport audit axe longitudinal ;
- le corps d'injecteur porte un pontet dont le fond et la face avant présentent une fente de réception dans laquelle une canule d'extrémité de la chambre de compactage et d'éjection vient se loger lorsque ladite chambre est en position de chargement ;
- le corps d'injecteur présente sur sa face supérieure une lumière dans laquelle peut s'engager le pêne arrière courbe porté par le moyen de verrouillage ;
- le corps d'injecteur est muni de deux appuis digitaux et de deux butées placées en avant des dits appuis ;
- le corps d'injecteur (ou la partie fixe de la chambre de compactage et d'éjection) est muni d'un organe d'accostage ou de retenue (ex : rainure) destiné à coopérer avec des moyens d'encliquetage disposés sur la chambre de compactage et d'éjection ;
- le poussoir comprend une partie proximale dont l'extrémité proximale est munie d'un appui digital, une partie intermédiaire de section plus faible et une tête de poussoir distale dont la section épouse celle de la cavité interne de la cartouche ;
- entre la tête du poussoir et l'ouverture proximale de la cartouche est interposé un pion en matériau déformable ;
- le bord supérieur de l'ouverture proximale de la chambre de compactage et d'éjection est muni de moyens d'encliquetage sous forme de languettes dont l'extrémité libre est en pointe de flèche, les barbillons de cette pointe de flèche coopérant avec la rainure du cavalier et avec un organe d'accostage ou de retenue de l'extrémité distale du corps d'injecteur lorsque la chambre de compactage et d'éjection est en position d'injection ; cette position de blocage étant irréversible, à ce stade, seul le mouvement du poussoir coulissant en vue du compactage et de l'injection est possible ;
- les faces latérales de la cartouche présentent des cliquets amovibles ou non (par exemple des bossages) pour le maintien en translation de la lentille intraoculaire ;
- la cartouche porte un élément en saillie (ex : une languette) qui est apte à coopérer avec la chambre de compactage et d'éjection lorsque celle-ci est en position d'injection afin de bloquer la cartouche en translation. L'élément en saillie bute contre une portion proximale de la partie distale de la chambre de compactage et d'éjection afin d'empêcher sa translation vers l'avant du dispositif, c'est-à-dire en éloignement du poussoir. L'élément saillant de la cartouche (par exemple agencé suivant l'axe longitudinal de la cartouche) lui sert également à se maintenir en position verticale dans un logement situé dans un récipient stérile destiné à renfermer ladite cartouche en attente d'être chargée dans le dispositif d'injection selon l'invention.

On notera que le récipient stérile présente une ouverture suffisamment large pour accueillir l'extrémité distale du dispositif avec la chambre de compactage et d'éjection agencée en position repliée contre le corps d'injecteur (dans une position latérale agencée suivant un axe parallèle à l'axe longitudinal du corps mais distinct de celui-ci) aux fins de chargement de la cartouche dans le corps suivant son axe longitudinal.

Selon un aspect qui peut être indépendant du dispositif décrit ci-dessus, l'invention porte également sur une cartouche telle qu'exposée brièvement ci-dessus et sur un récipient stérile pour le stockage d'une telle cartouche.

On notera que l'invention concerne également un ensemble d'injection qui comprend le dispositif d'injection tel qu'exposé ci-dessus (ce dispositif comprend le corps d'injecteur logeant le poussoir et la chambre de compactage et d'éjection) et une cartouche recevant une lentille intraoculaire (implant). Les caractéristiques visées ci-dessus s'appliquent également à l'ensemble d'injection.

D'autres caractéristiques et avantages de l'invention ressortiront de la description qui va suivre d'exemples de réalisation non limitatifs de l'invention, en référence aux dessins annexés dans lesquels :
- La Figure la est une vue générale en perspective de l'avant d'un dispositif d'injecteur de lentille intraoculaire souple conforme à l'invention avant verrouillage de la chambre de compactage et d'éjection ;
- La Figure 1b est une vue générale de profil du dispositif d'injecteur de la Figure 1a après verrouillage de la chambre de compactage et d'éjection ;
- La Figure 2a est une vue analogue à la Figure la la chambre de verrouillage et d'éjection étant ouverte et la cartouche contenant la lentille intraoculaire étant chargée dans la chambre de réception de la partie distale du corps de l'injecteur ;
   La Figure 2b est une vue générale de profil du dispositif d'injecteur de la Figure 2a ;
- La Figure 3 est une vue en perspective éclatée du poussoir et de la cartouche en position d'alignement à l'intérieur du corps d'injecteur ;
- La Figure 4 est une vue en perspective agrandie du moyen de verrouillage alternatif porté par le corps d'injecteur ;
- La Figure 5a est une vue en perspective agrandie de la chambre de compactage et d'éjection en position ouverte sans cartouche ;
- La Figure 5b est une vue en perspective agrandie de la chambre de compactage et d'éjection en position ouverte, la position relative de la cartouche par rapport à cette chambre, lorsqu'elle est tenue par le corps d'injecteur, étant montrée;
- La Figure 5c est une vue en perspective agrandie de la chambre de compactage et d'éjection en position fermée.
- La Figure 6a est une vue en perspective agrandie de la cartouche sans lentille intraoculaire;
- La Figure 6b est une vue en perspective agrandie du fond de la cartouche ;
- La Figure 6c est une vue en plan de dessus agrandie du fond de la cartouche, illustrant la position de stockage sans contrainte d'une lentille intraoculaire ;
- La Figure 7a est une vue en perspective de dessus d'un récipient de stockage de la cartouche ;
- La Figure 7b est une vue en perspective de dessus illustrant l'introduction du dispositif d'injecteur dans l'emballage de la Figure 7a pour le chargement de la cartouche.
- La Figure 8 est une vue en perspective éclatée des éléments constitutifs d'un ensemble d'injection conforme à l'invention, la cartouche étant représentée en condition ouverte et en condition fermée.

Dans les différentes figures, les éléments identiques ou fonctionnellement analogues sont indiqués par les mêmes références.

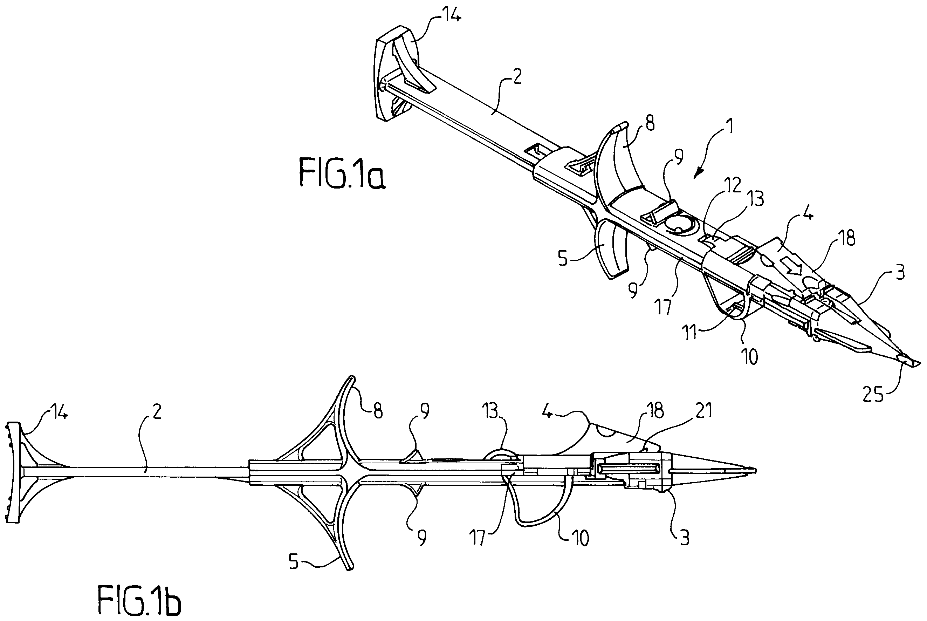

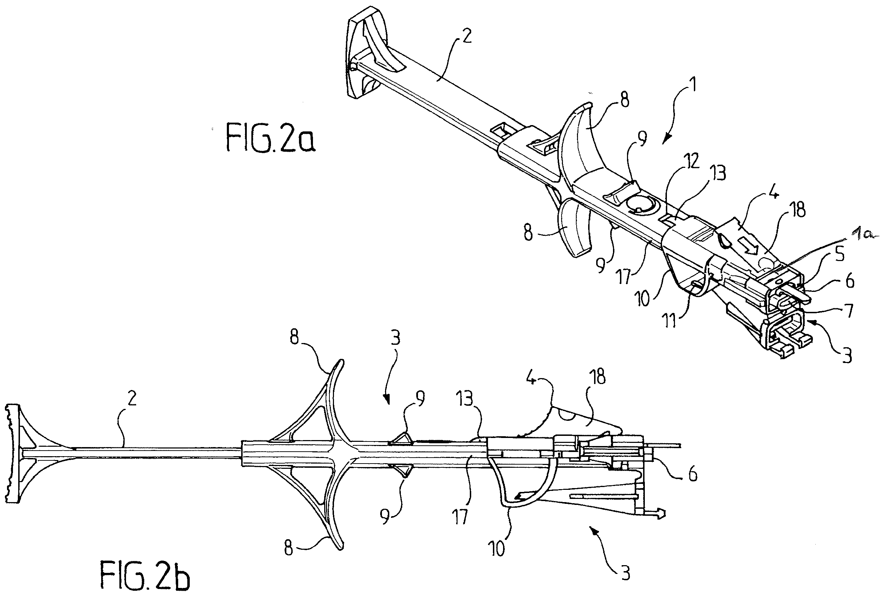

Sur les Figures 1a, 1b, 2a et 2b on voit que l'injecteur est constitué d'un corps d'injecteur 1, d'un poussoir 2 engagé dans le corps d'injecteur 1 à l'extrémité proximale de ce dernier, d'une chambre de compactage et d'éjection 3 et d'un verrou 4. La chambre de compactage et d'éjection est montée à rotation sur l'extrémité distale du corps d'injecteur 1 de manière à pouvoir être repliée le long de ce dernier pour dégager l'accès à une chambre de réception 5 (Figure 2a) conformée pour accueillir une cartouche 6 contenant une lentille intraoculaire 7 à l'état non contraint. Le corps d'injecteur 1 est muni de deux appuis digitaux 8 et de deux butées 9 placées en avant des dits appuis afin d'assurer une prise en main sûre et aisée pour l'utilisateur, sans risque que celui-ci n'appuie sur le poussoir de manière intempestive. Cette disposition est particulièrement utile lors des manipulations nécessaires à la mise en place de la cartouche dans l'injecteur. Le corps d'injecteur 1 porte également un pontet 10 dont le fond et la face avant présentent une fente de réception dans laquelle la canule d'extrémité 25 de la chambre de compactage et d'éjection 3 vient se loger lorsque ladite chambre est basculée en position ouverte (position de chargement d'une cartouche) comme on le voit sur les Figures 2a et 2b. Le corps d'injecteur 1 présente sur sa face supérieure une lumière 12 dans laquelle peut s'engager un pêne arrière courbe 13 porté par le verrou 4. Alors que le corps d'injecteur 1 et le poussoir 2 sont, de préférence, réalisés dans un matériau synthétique tel que le polycarbonate ou l'ABS, la chambre de compactage et d'éjection 3 et la cartouche 6 sont réalisées, de préférence, dans un matériau polymère compatible avec une stérilisation en phase vapeur tel que le polypropylène.

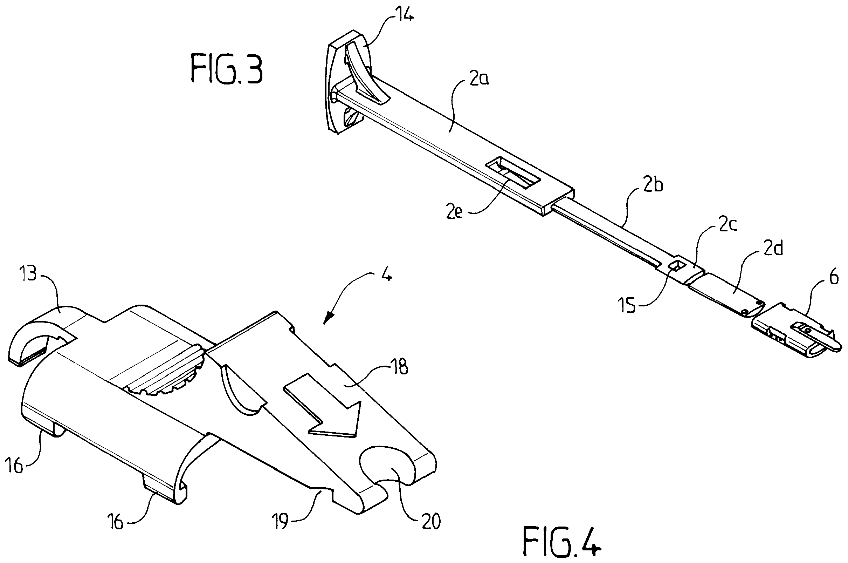

Sur la Figure 3 on voit que le poussoir 2 comprend une partie proximale 2a dont l'extrémité proximale est munie d'un appui digital 14, d'une partie intermédiaire 2b de section plus faible et d'une tête de poussoir 2c dont la section épouse celle de la cavité interne de la cartouche 6. La tête de poussoir 2c présente sur sa face supérieure une creusure ou une lumière 15 destinée à recevoir le pêne arrière courbe du verrou 4. Dans l'exemple de réalisation ici représenté, entre la tête 2c du poussoir 2 et l'ouverture proximale de la cartouche 6 est interposé un pion 2d en matériau déformable destiné à transmettre la poussée du poussoir à la lentille intraoculaire 7 sans risque de l'endommager. Le pion 2d est de section semblable à celle de la cavité interne de la cartouche 6. La partie proximale 2a du poussoir 2 est munie d'un cliquet 2e destiné à empêcher un dégagement intempestif du poussoir2 par rapport au corps d'injecteur 1.

Sur la Figure 4 on voit que le moyen de verrouillage alternatif 4 présente la forme générale d'un cavalier muni de pattes 16 destinées à guider le coulissement dudit cavalier dans des rainures latérales 17 aménagées sur les cotés longitudinaux du corps d'injecteur 1. La partie distale du cavalier 4 porte une languette 18 qui s'étend en saillie vers l'avant. La languette 18 bloque la chambre de compactage et d'éjection 3 lorsque cette dernière est en position fermée (position d'injection). L'arrière de la languette 18 est épaissi de manière à former un appui digital facilitant le maniement du cavalier vers sa position de verrouillage de la chambre de compactage et d'éjection 3. Le coulissement du cavalier 4 vers sa position de blocage de la chambre de compactage et d'éjection 3 dégage le pêne courbe 13 de l'ouverture 15 dans la tête 2c du poussoir 2, permettant ainsi le déplacement dudit poussoir. Près de son extrémité distale, la face inférieure de la languette 18 comporte une rainure 19 destinée à coopérer avec des moyens d'encliquetage 21 disposés sur la chambre de compactage et d'éjection 3. Le bord de délimitation avant de la languette 18 présente une ouverture 20 pour le passage d'une aiguille d'injection d'un produit lubrifiant viscoélastique biocompatible dans la chambre de compactage et d'éjection 3.

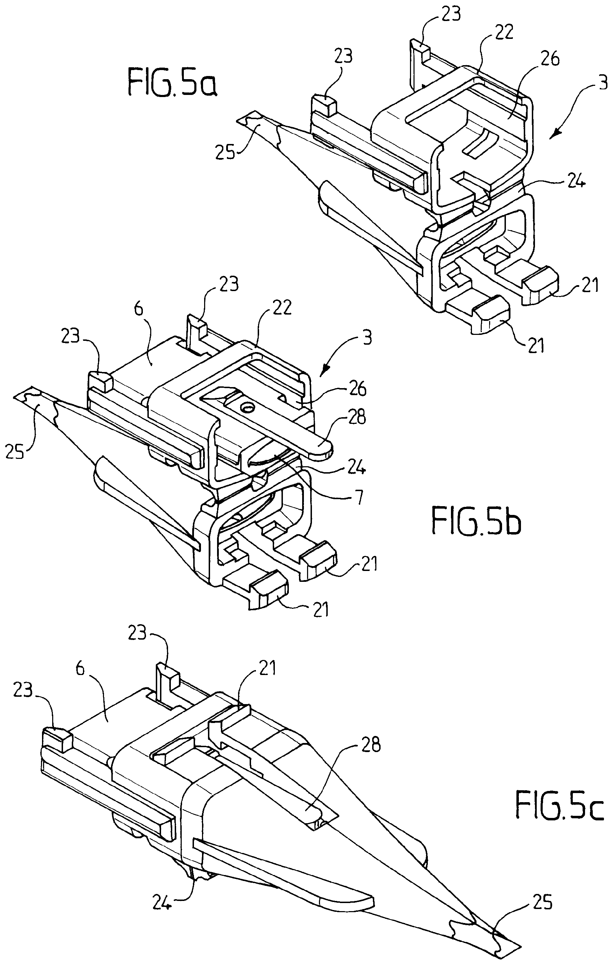

Sur les Figures 5a et 5b la chambre de compactage et d'éjection 3 est représentée en position ouverte (position de chargement). Elle comprend une partie proximale fixe telle qu'une monture 22 destinée à s'adapter par friction sur l'extrémité distale du corps d'injecteur 1. Deux cliquets 23 assurent la tenue de la monture 22 par engagement dans des rainures prévues à cet effet dans les parois latérales externes du corps d'injecteur 1. La chambre de compactage et d'éjection 3 comprend une partie distale fonctionnelle (servant au compactage et à l'injection d'une lentille) qui est articulée à la monture 22 par une charnière souple 24 dont l'axe est orthogonal par rapport à l'axe longitudinal du corps d'injection 1 et décalé transversalement par rapport audit axe longitudinal (axe horizontal situé en dessous du corps 1). La partie distale de la chambre de compactage et d'éjection 3 est conformée en un entonnoir se raccordant à une canule de sortie 25. Les faces latérales internes de la monture 22 sont pourvues de rainures 26 pour renforcer le maintien par friction de la monture 22 sur l'extrémité distale du corps d'injecteur 1. Le bord supérieur de l'ouverture proximale de la partie distale de la chambre de compactage et d'éjection 3 est muni de moyens d'encliquetage 21, sous forme de languettes dont l'extrémité libre est en pointe de flèche, les barbillons de cette pointe de flèche coopérant, d'une part, pour les ergots supérieurs avec la rainure 19 du cavalier 4 (rainure pratiquée dans la face inférieure du cavalier) et, d'autre part, pour les ergots inférieurs avec un organe d'accostage ou de retenue la (figure 2a) de l'extrémité distale du corps d'injecteur 1 lorsque la chambre de compactage et d'éjection 3 est fermée (on notera que l'organe d'accostage ou de retenue peut alternativement être prévu sur la partie fixe de la chambre 3 telles que la monture 22). On obtient ainsi un verrouillage positif de la partie distale (mobile) de la chambre de compactage et d'éjection 3, d'abord sur le corps d'injecteur 1 par encliquètement sur l'organe d'accostage ou de retenue 1a, puis avec le cavalier 4 lorsque ce dernier est déplacé.

Lorsque le cavalier 4 est poussé en avant, celui-ci retire le pêne courbe 13 de la rainure 15. Le mouvement du poussoir 2 qui était jusque là impossible du fait de la présence du pêne dans la rainure, n'est ainsi possible que lorsque le cavalier 4 a effectivement été mis en position de blocage de la chambre de compression et d'injection en position fermée.

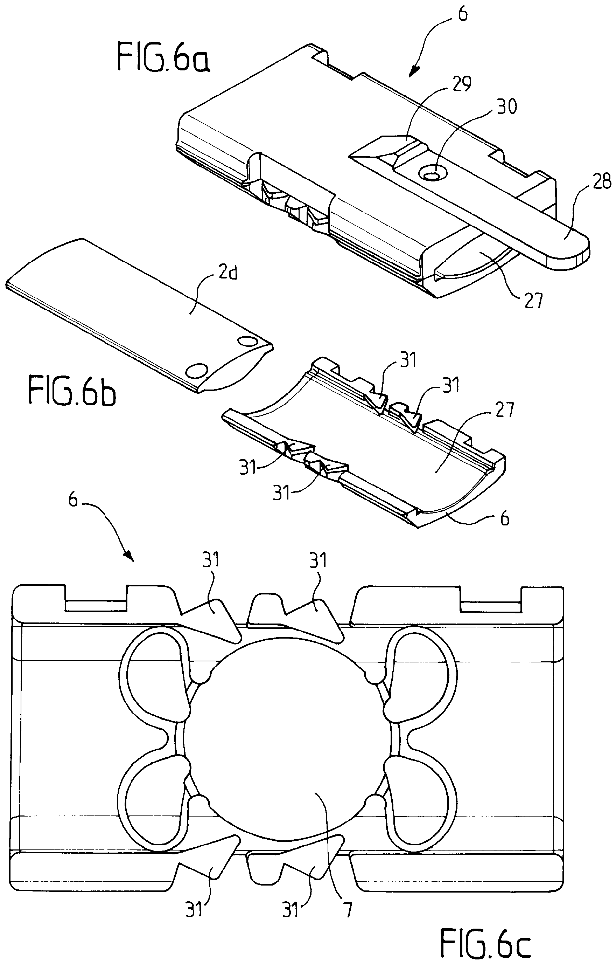

Sur les Figures 6a, 6b et 6c, on voit que la cartouche 6 est de forme générale parallélépipédique et que ses parois internes définissent une cavité de réception 27 pour une lentille intraoculaire 7. La face supérieure de la cartouche 6 porte un élément en saillie tel qu'une languette en saillie 28 dont l'extrémité arrière forme un crochet 29. Près du crochet 29 un perçage 30 permet le passage d'une aiguille pour l'injection d'un lubrifiant viscoélastique biocompatible dans la cavité 27. La languette 28 à une double fonction : elle maintient la cartouche 6 en position verticale dans son récipient de stockage et elle s'engage sur la partie proximale de la partie distale de la chambre de compactage et d'éjection 3 pour bloquer la cartouche en translation lorsque la chambre de compactage et d'éjection est en position fermée (position d'éjection). De manière avantageuse, comme représenté aux figures 6b et 6c, les faces latérales de la cartouche 6 présentent des cliquets 31 qui assurent un maintien en translation de l'implant 7. De préférence les cliquets 31 sont venus de moulage avec la cartouche 6. Alternativement, les cliquets amovibles peuvent être remplacés par des bossoirs (fixes).

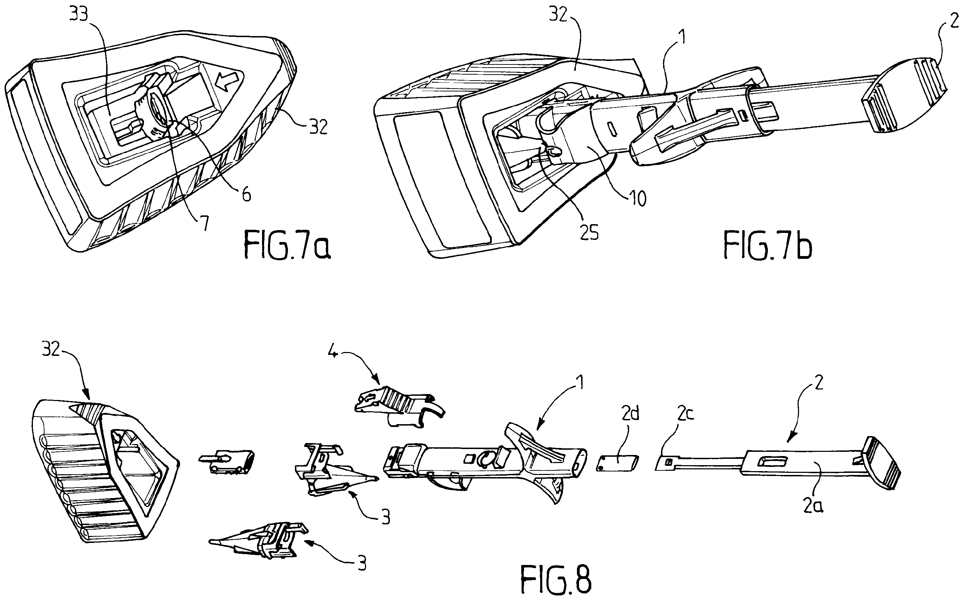

Les Figures 7a et 7b montrent un exemple de réalisation d'un emballage ou récipient stérile spécialement conçu pour le stockage d'une cartouche 6 et son insertion dans le corps d'injecteur 1 au moment de son utilisation. On voit que cet emballage 32 présente un large puits 33 dans lequel la cartouche 6 à l'intérieur de laquelle est logée une IOL 7 est maintenue en position verticale sensiblement au centre du puits 33. De part et d'autre de la cartouche 6 un espace suffisant est ménagé pour recevoir l'extrémité distale de l'injecteur en position ouverte, la chambre de compactage et d'injection 3 étant repliée le long du corps d'injecteur 1. On comprend que la cartouche 6 peut ainsi être maintenue stockée immergée dans un milieu aqueux salin et maintenue stérile jusqu'au moment de son utilisation par la fermeture de l'emballage 32, par exemple par un opercule détachable. Lors de l'utilisation, l'ensemble selon l'invention permet au chirurgien d'être prêt à injecter l'IOL en exécutant seulement trois mouvements sans aucun risque d'endommager l'IOL, d'affecter son état stérile ou de se mouiller les doigts: enfoncement de l'injecteur sur la cartouche pour le chargement de celle-ci, fermeture de la chambre de compactage et d'éjection, verrouillage de la chambre de compactage et d'éjection en position fermée avec libération simultanée du poussoir.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif d'injection pour lentille intraoculaire souple comprenant un corps d'injecteur (1) logeant un poussoir coulissant (2) et une chambre de compactage et d'éjection (3) solidaire dudit corps d'injecteur, **caractérisé en ce que** la chambre de compactage et d'injection (3,22) est articulée sur l'extrémité distale du corps d'injecteur (1) de manière à pouvoir prendre deux positions, d'une part, une position de chargement dans laquelle au moins une partie de la chambre de compactage et d'injection est repliée le long du corps d'injecteur de manière décalée transversalement par rapport à l'axe longitudinal du corps d'injecteur (1) de façon à laisser libre la place dans le corps d'injecteur, suivant ledit axe longitudinal, pour y loger une cartouche (6) contenant une lentille intraoculaire (7) à l'état non contraint et, d'autre part, une position d'injection dans laquelle la chambre de compactage et d'injection (3) est verrouillée dans le prolongement dudit corps d'injecteur.

2. Dispositif d'injection selon la revendication 1, **caractérisé en ce que** la partie distale du corps d'injecteur (1) comporte une chambre de réception (5) pour la cartouche (6) et **en ce que** le corps d'injecteur (1) porte un moyen de verrouillage (4) qui est apte à occuper deux positions de blocage successives, à savoir une première position qui bloque le mouvement du poussoir (2) lorsque la chambre de compactage et d'injection (3) est dans la position de chargement d'une cartouche, puis une deuxième position qui bloque la chambre de compactage et d'éjection (3) en position d'injection dans le prolongement du corps d'injecteur (1) et libère le poussoir (2).

3. Dispositif d'injection selon la revendication 2, **caractérisé en ce que** le moyen de verrouillage (4) est apte à coulisser le long du corps d'injecteur (1) pour passer de façon irréversible de la première position de blocage à la deuxième.

4. Dispositif d'injection selon la revendication 2 ou 3, **caractérisé en ce que** le moyen de verrouillage (4) comprend une portion de préhension pour un utilisateur et deux portions (13, 18) disposées aux deux extrémités opposées proximale et distale dudit moyen de verrouillage et qui sont respectivement aptes à coopérer avec le poussoir coulissant (2) et la chambre de compactage et d'éjection (3).

5. Dispositif d'injection selon l'une quelconque des revendications 2 à 4, **caractérisé en ce que** le moyen de verrouillage (4) présente la forme générale d'un cavalier muni de pattes (16) destinées à guider le coulissement dudit cavalier dans des rainures latérales (17) aménagées sur les côtés longitudinaux du corps d'injecteur (1).

6. Dispositif d'injection selon la revendication 5, **caractérisé en ce que** le cavalier porte à sa partie proximale un pêne courbe (13) coopérant avec une ouverture (15) du poussoir (2).

7. Dispositif d'injection selon la revendication 5 ou 6 **caractérisé en ce que** la partie distale du cavalier (4) porte une languette (18) qui s'étend en saillie vers l'avant et est destinée à s'engager, dans la deuxième position de blocage, sur une portion proximale (22) de la chambre de compactage et d'éjection (3) pour bloquer cette dernière en position fermée.

8. Dispositif d'injection selon la revendication 7, **caractérisé en ce que** près de son extrémité distale, la face inférieure de la languette (18) du cavalier (4) comporte une rainure (19) destinée à coopérer avec des moyens d'encliquetage (21) disposés sur la chambre de compactage et d'éjection (3).

9. Dispositif d'injection selon l'une des revendications 1 à 8 , **caractérisé en ce que** la chambre de compactage et d'éjection (3) comporte une partie proximale (22) montée de manière fixe sur le corps d'injecteur (1) et une partie distale qui est articulée par une charnière (24) à la partie proximale (22), l'axe de la charnière étant orthogonal par rapport à l'axe longitudinal du corps d'injecteur (1) et décalé transversalement par rapport audit axe longitudinal.

10. Dispositif d'injection selon l'une quelconque des revendications 1 à 9, **caractérisé en ce que** le corps d'injecteur (1) porte un pontet (10) dont le fond et la face avant présentent une fente de réception (11) dans laquelle une canule d'extrémité (25) de la chambre de compactage et d'éjection (3) vient se loger lorsque ladite chambre est en position de chargement.

11. Dispositif d'injection selon la revendication 6 **caractérisé en ce que** le corps d'injecteur (1) présente sur sa face supérieure une lumière (12) dans laquelle peut s'engager le pêne arrière courbe (13) porté par le moyen de verrouillage (4).

12. Dispositif d'injection selon la revendication 10 ou 11, **caractérisé en ce que** le corps d'injecteur (1) est muni de deux appuis digitaux (8) et de deux butées (9) placées en avant des dits appuis (8).

13. Dispositif d'injection selon l'une quelconque des revendications 8 à 12, **caractérisé en ce que** le bord supérieur de l'ouverture proximale de la chambre de compactage et d'éjection (3) est muni de moyens d'encliquetage (21) sous forme de languettes dont l'extrémité libre est en pointe de flèche, les barbillons de cette pointe de flèche coopérant avec la rainure (19) du cavalier (4) et avec un organe d'accostage de l'extrémité distale du corps d'injecteur (1) lorsque la chambre de compactage et d'éjection (3) est en position d'injection.

14. Ensemble d'injection pour lentille intraoculaire, **caractérisé en ce qu'**il comprend un dispositif d'injection selon l'une des revendications 1 à 13 et une cartouche (6) contenant une lentille intraoculaire (7) à l'état non contraint.

15. Ensemble d'injection selon la revendication 14, **caractérisé en ce que** la cartouche (6) porte un élément en saillie (28) qui est apte à coopérer avec la chambre de compactage et d'éjection (3) lorsque celle-ci est en position d'injection afin de bloquer la cartouche en translation.

## Patentansprüche

1. Vorrichtung zum Einsetzen einer flexiblen Intraokularlinse, umfassend einen Einsatzkörper (1), der einen gleitenden Schieber (2) und eine Kompaktierungs- und Ausstoßkammer aufnimmt, die fest mit dem Einsatzkörper verbunden ist, **dadurch gekennzeichnet, dass** die Kompaktierungs- und Einsatzkammer (3, 22) auf dem distalen Ende des Einsatzkörpers (1) gelenkig verbunden ist, um zwei Positionen einnehmen zu können, einerseits eine Ladeposition, in der mindestens ein Teil der Kompaktierungs- und Einsatzkammer entlang des Einsatzkörpers quer versetzt mit Bezug auf die Längsachse des Einsatzkörpers (1) derart umgeknickt ist, dass der Platz im Einsatzkörper gemäß der Längsachse frei gelassen wird, so dass dort eine Patrone (6) aufgenommen wird, die eine Intraokularlinse (7) im nicht gespanntem Zustand enthält, andererseits eine Einsatzposition, in der die Kompaktierungs- und Einsatzkammer (3) in der Verlängerung des Einsatzkörpers verriegelt ist.

2. Vorrichtung zum Einsetzen nach Anspruch 1, **dadurch gekennzeichnet, dass** der distale Teil des Einsatzkörpers (1) eine Aufnahmekammer (5) für die Patrone (6) umfasst, und dadurch, dass der Einsatzkörper (1) ein Verriegelungsmittel (4) trägt, das dazu in der Lage ist, zwei aufeinanderfolgende Blockierpositionen einzunehmen, d. h. eine erste Position, die die Bewegung des Schiebers (2) blockiert, wenn sich die Kompaktierungs- und Einsatzkammer (3) in der Ladeposition einer Patrone befindet, dann eine zweite Position, die die Kompaktierungs- und Ausstoßkammer (3) in der Einsatzposition in der Verlängerung des Einsatzkörpers (1) blockiert und den Schieber (2) freisetzt.

3. Einsatzvorrichtung nach Anspruch 2, **dadurch gekennzeichnet, dass** das Verriegelungsmittel (4) dazu in der Lage ist, entlang dem Einsatzkörper (1) zu gleiten, um auf unumkehrbare Weise von der ersten in die zweite Blockierposition überzugehen.

4. Einsatzvorrichtung nach Anspruch 2 oder 3, **dadurch gekennzeichnet, dass** das Verriegelungsmittel (4) einen Vorspannungsabschnitt für einen Benutzer und zwei Abschnitte (13, 18) umfasst, die an den zwei gegenüberliegenden Enden, proximal und distal, des Verriegelungsmittels angeordnet sind, und die jeweils dazu in der Lage sind, mit dem gleitenden Schieber (2) und der Kompaktierungs- und Ausstoßkammer (3) zusammenzuarbeiten.

5. Vorrichtung zum Einsetzen nach einem der Ansprüche 2 bis 4, **dadurch gekennzeichnet, dass** das Verriegelungsmittel 4 die allgemeine Form einer Klammer, ausgestattet mit Füßen (16), darstellt, die ausgelegt sind, um das Gleiten der Klammer in den seitlichen Nuten (17) zu führen, die auf den Längsseiten des Einsatzkörpers (1) angebracht sind.

6. Vorrichtung zum Einsetzen nach Anspruch 5, **dadurch gekennzeichnet, dass** die Klammer an ihrem proximalen Teil einen gekrümmten Riegel (13) trägt, der mit einer Öffnung (15) des Schiebers (2) zusammenarbeitet.

7. Vorrichtung zum Einsetzen nach Anspruch 5 oder 6, **dadurch gekennzeichnet, dass** der distale Teil der Klammer (4) eine Lasche (18) trägt, die sich vorspringend nach vorne erstreckt und ausgelegt ist, um in der zweiten Blockierposition auf einem proximalen Abschnitt (22) der Kompaktierungs- und Ausstoßkammer (3) einzugreifen, um diese Letztere in der geschlossenen Position zu blockieren.

8. Vorrichtung zum Einsetzen nach Anspruch 7, **dadurch gekennzeichnet, dass** nahe an ihrem distalen Ende die untere Seite der Lasche (18) der Klammer (4) eine Nut (19) umfasst, die ausgelegt ist, um mit Schnappmitteln (21) zusammenzuarbeiten, die auf der Kompaktierungs- und Ausstoßkammer (3) angeordnet sind.

9. Vorrichtung zum Einsetzen nach einem der Ansprüche 1 bis 8, **dadurch gekennzeichnet, dass** die Kompaktierungs- und Ausstoßkammer (3) einen proximalen Teil (22) umfasst, der auf feste Weise auf dem Einsatzkörper (1) montiert ist, und einen distalen Teil, der durch ein Scharnier (24) mit dem proximalen Teil (22) gelenkig verbunden ist, wobei die Achse des Scharniers orthogonal mit Bezug auf die Längsachse des Einsatzkörpers (1) und quer versetzt mit Bezug auf die Längsachse ist.

10. Vorrichtung zum Einsetzen nach einem der Ansprüche 1 bis 9, **dadurch gekennzeichnet, dass** der Einsatzkörper (1) eine Brücke (10) umfasst, deren Boden und vordere Seite einen Aufnahmeschlitz (11) aufweisen, in den eine Endkanüle (25) der Kompaktierungs- und Ausstoßkammer (3) aufgenommen wird, wenn sich die Kammer in der Ladeposition befindet.

11. Vorrichtung zum Einsetzen nach Anspruch 6, **dadurch gekennzeichnet, dass** der Einsatzkörper (1) auf seiner oberen Fläche eine Öffnung (12) aufweist, in die der hintere gekrümmte Riegel (13) eingreifen kann, der vom Verriegelungsmittel (4) getragen wird.

12. Vorrichtung zum Einsetzen nach Anspruch 10 oder 11, **dadurch gekennzeichnet, dass** der Einsatzkörper (1) mit zwei digitalen Auflagen (8) und zwei Anschlägen (9) ausgestattet ist, die vor den Anschlägen (8) platziert sind.

13. Vorrichtung zum Einsetzen nach einem der Ansprüche 8 bis 12, **dadurch gekennzeichnet, dass** der obere Rand der proximalen Öffnung der Kompaktierungs- und Ausstoßkammer (3) mit Schnappmitteln (21) in Form von Laschen ausgestattet ist, deren freies Ende die Form einer Pfeilspitze aufweist, wobei die Haken dieser Pfeilspitze mit der Nut (19) der Klammer (4) und mit einem Anordnungsorgan des distalen Endes des Einsatzkörpers (1) zusammenarbeiten, wenn sich die Kompaktierungs- und Ausstoßkammer (3) in der Einsatzposition befindet.

14. Einheit zum Einsetzen für Intraokularlinse, **dadurch gekennzeichnet, dass** sie eine Vorrichtung zum Einsetzen nach einem der Ansprüche 1 bis 13 und eine Patrone (6) umfasst, enthaltend eine Intraokularlinse (7) im nicht gespannten Zustand.

15. Einheit zum Einsetzen nach Anspruch 14, **dadurch gekennzeichnet, dass** die Patrone (6) ein vorspringendes Element (28) umfasst, das ausgelegt ist, um mit der Kompaktierungs- und Ausstoßkammer (3) zusammenzuarbeiten, wenn sich diese in der Einsatzposition befindet, um die Patrone in Translation zu blockieren.

## Claims

1. An injection device for a flexible intraocular lens, comprising an injector body (1) accommodating a sliding pusher (2), and a compaction and ejection chamber (3) rigidly connected to said injector body, **characterized in that** the compaction and injection chamber (3,22) is articulated on the distal end of the injector body (1) so as to be able to adopt two positions, one loading position in which at least part of the compaction and injection chamber is folded along the injector body in a transversally offset manner relative to the longitudinal axis of the injector body (1) so as to leave free room in the injector body, along said longitudinal axis, to accommodate therein a cartridge (6) containing an intraocular lens (7) in the non-stressed state, and an injection position in which the compaction and injection chamber (3) is locked in the continuation of said injector body.

2. The injection device as claimed in claim 1, **characterized in that** the distal part of the injector body (1) has a reception chamber (5) for the cartridge (6), and **in that** the injector body (1) carries a locking means (4) which is suitable for occupying two successive blocking positions, namely a first position which blocks the movement of the pusher (2) when the compaction and injection chamber (3) is in the position for loading a cartridge and a second position which blocks the compaction and ejection chamber (3) in the injection position in the continuation of the injector body and frees the pusher (2).

3. The injection device as claimed in claim 2, **characterized in that** the locking means is suitable for sliding along the injector body (1) to change in an irreversible manner from the first blocking position to the second one.

4. The injection device as claimed in claim 2 or 3, **characterized in that** the locking means (4) comprises a handling portion for a user and two portions (13, 18) disposed at the two opposed proximal and distal ends of said locking means and which are respectively suitable for cooperating with the sliding pusher (2) and the compaction and injection chamber (3).

5. The injection device as claimed in any one of claims 2 through 4, **characterized in that** the locking means (4) has the general form of a rider provided with tabs (16) that are intended to guide the sliding of said rider in lateral grooves (17) formed on the longitudinal sides of the injector body (1).

6. The injection device as claimed in claim 5, **characterized in that** the rider carries, at its proximal part, a curved bolt (13) cooperating with an opening (15) of the pusher (2).

7. The injection device as claimed in claim 5 or 6, **characterized in that** the distal part of the rider (4) carries a tongue (18) which projects forward and, in the second blocking position, is intended to engage on a proximal portion of the compaction and ejection chamber (3) in order to block the latter in the closed position.

8. The injection device as claimed in claim 7, **characterized in that**, near its distal end, the lower face of the tongue (18) of the rider (4) has a groove (19) intended to cooperate with snap-fitting means (21) arranged on the compaction and ejection chamber (3).

9. The injection device as claimed in any one of claims 1 through 8, **characterized in that** the compaction and ejection chamber (3) comprises a proximal end (22) that is fixed onto the injection body (1) and a distal end that is articulated on the proximal end (22) via a hinge (24), the axis of the hinge being orthogonal with respect to the longitudinal axis of the injection body (1) and transversally offset relative to said longitudinal axis.

10. The injection device as claimed in any one of claims 1 through 9, **characterized in that** the injector body (1) carries a bow (10), of which the bottom and the front face have a receiving slit (11) in which the end cannula (25) of the compaction and ejection chamber (3) is accommodated when said chamber is in the loading position.

11. The injection device as claimed in claim 6, **characterized in that** the injector body (1) has, on its upper face, a slot (12) in which the curved rear bolt (13) carried by the locking means (4) can engage.

12. The injection device as claimed in claim 10 or 11, **characterized in that** the injector body (1) is provided with two finger supports (8) and with two abutments (9) placed in front of said supports (8).

13. The injection device as claimed in any one of claims 8 through 12, **characterized in that** the upper edge of the proximal opening of the compaction and ejection chamber (3) is provided with snap-fitting means (21) in the form of tongues, of which the free end is shaped like an arrow head, the barbs of this arrow head cooperating with the groove (19) of the rider (4) and with an opening in the distal end of the injector body (1) when the compaction and ejection chamber (3) is in the injection position.

14. An injection assembly for a intraocular, **characterized in that** it comprises an assembly device as claimed in any one of claims 1 through 13 and a cartridge (6) containg an intraocular lens (7) in the non stressed state.

15. The injection assembly as claimed in claim 14, **characterized in that** the cartridge (6) carries a protruding member (28) that is suitable for cooperating with the compaction and ejection chamber (3) when the latter is in the injection position in order to block the cartridge in translation.
